# EUROPEAN PATENT APPLICATION

(11) **EP 1 398 380 A1**
(43) Date of publication of application: **17.03.2004**
(21) Application number: 02022252.7
(22) Date of filing: 02.10.2002
(51) Int. Cl.: C12N 15/39, C12N 15/09, C12N 15/63, C07K 7/06, C12N 5/14, C12N 5/16, C12N 5/22, G01N 33/53, A61K 39/295

(54) **Orthopoxvirus antigens and use thereof**

(30) Priority: 12.09.2002 DE 10242318
(71) Applicant: GSF-Forschungszentrum für Umwelt und Gesundheit GmbH, 80807 Oberschleissheim (DE)
(72) Inventor: Drexler, Ingo, 80797 München (DE); Erfle, Volker, 81679 München (DE); Staib, Caroline, 80804 München (DE); Sutter, Gerd, 80803 München (DE)
(74) Representative: Reinhard - Skuhra - Weise & Partner

(57) **Abstract**

The present invention is directed to a method of detecting a T cell response induced by smallpox vaccines or Orthopoxvirus vector vaccines in a mammal and to immunogenic Orthopoxvirus antigens and the nucleic acids encoding same. The present invention further relates to pharmaceutical compositions and their use in the protective immunization against a smallpox infection.

## Description

The present invention is directed to a method of detecting a T cell response induced by smallpox vaccines or Orthopoxvirus vector vaccines in a mammal and to immunogenic Orthopoxvirus antigens and the nucleic acids encoding same. The present invention further relates to pharmaceutical compositions and their use in the protective immunization against a smallpox infection.

Despite worldwide eradication of naturally occurring variola virus, smallpox remains a potential threat to both civilian and military populations. New safe smallpox vaccines are being developed, and there is an urgent need for methods to evaluate vaccine efficacy following immunization.

The anthrax attacks in the United States changed a theoretical scenario of bioterrorism into a realistic threat. Variola virus, the cause of human smallpox, emerged as one of the infectious agents being listed with highest priority for research on prevention and therapy (1). Its malevolent use was predicted to result in a rapid epidemic in human populations because little immunity exists since vaccination with the *Orthopoxvirus* relative (vaccinia virus) has ceased (2). Vaccinia virus belongs to the genus Orthopoxvirus of the family of poxviruses.

Certain strains of vaccinia virus have been used for many years as live vaccine to immunize against smallpox, for example the Elstree strain of the Lister Institute in the UK. Because of the complications which may derive from the vaccination (Schär, Zeitschr. für Praventivmedizin 18, 41-44 [1973]), and since the declaration in 1980 by the WHO that smallpox had been eradicated nowadays only people at high risk are vaccinated against smallpox.

Vaccinia viruses have also been used as vectors for production and delivery of foreign antigens (Smith et al., Biotechnology and Genetic Engineering Reviews 2, 383-407 [1984]). This entails DNA sequences (genes) which code for foreign antigens being introduced, with the aid of DNA recombination techniques, into the genome of the vaccinia viruses. If the gene is integrated at a site in the viral DNA which is non-essential for the life cycle of the virus, it is possible for the newly produced recombinant vaccinia virus to be infectious, that is to say able to infect foreign cells and thus to express the integrated DNA sequence (EP Patent Applications No. 83, 286 and No. 110, 385). The recombinant vaccinia viruses prepared in this way can be used, on the one hand, as live vaccines for the prophylaxis of infections, on the other hand, for the preparation of heterologous proteins in eukaryotic cells.

The efficacy of *in vivo* produced replication-competent vaccinia virus as live viral vaccine was impressively demonstrated by the worldwide eradication of naturally occurring variola virus (3). Yet because of the adverse events associated with vaccinia virus vaccination, safer second-generation vaccines which are produced in tissue culture are an urgent need (4). However, this need is accompanied with the scientific challenge to determine vaccine efficacy in the absence of the naturally occurring smallpox disease, and the question of vaccine safety in individuals with immunodeficiencies caused by HIV infection or iatrogenic immunosuppression (5).

In fact, because smallpox vaccination ended more than 20 years ago, there is only limited knowledge about immunologic correlates of protective vaccination. The high degree of sequence conservation among viral proteins encoded by *Orthopoxvirus* genomes correlates well with the possibility for genus-wide cross-reacting protective immune responses. While there are means to monitor induced antibodies by virus neutralization, hemagglutination-inhibition, complement-fixation, or ELISA after vaccination of humans, cell-mediated immunity has been scarcely assessed (for review (6)). Yet, experience with individual human vaccinees and work in animal models suggest the vital role of cell-mediated immune responses in protection (7, 8). The long-lasting memory of cellular immunity is demonstrated by virus-specific cytotoxic T cells (CTL) being detectable even decades after primary vaccination (9).

Immune recognition by most T lymphocytes occurs through surface interactions involving a specific T cell receptor (TCR) on the lymphocyte and an antigen on the target cell. Unlike antibodies, TCR do not recognize intact antigens. With few exceptions, they only recognize fragments of antigens, in the form of peptides, which are presented on the cell surface by molecules of the major histocompatibility complex (MHC). The ability of T cells to recognize antigen only when it is presented by MHC molecules is called "MHC restriction" . The reacting T cells are referred to as MHC-restricted lymphocytes. There are two classes of MHC molecules, class I and class II, which are functionally distinguished by the type of antigen they bind and the subset of T cells with which they interact. The dichotomy between class I and class II molecules relates to their different roles in T cell activation. Class I molecules present peptides to MHC-restricted CD8 positive cytotoxic T lymphocytes (CTL). These cells directly lyse target cells; therefore it is biologically appropriate that they recognize peptides derived from intracellular antigens that are presented by class I molecules. Moreover, since class I molecules are expressed by nearly all nucleated cells, CTL are able to recognize and destroy virtually any cell if it presents the appropriate MHC/peptide complex. On the other hand, class II molecules present peptides to CD4 positive T helper cells, the primary function of which is to secrete cytokines that promote activities of other lymphocytes, including B cells, macrophages and CTL. They play a dominant role in orchestrating an immune response.

However, substantial information about relevant *Orthopoxvirus* antigens and eptitope specificities of virus-specific CTL responses is still lacking.

Therefore, it is an object of the present invention to provide new *Orthopoxvirus* antigens, which can be used in the detection of immune responses against smallpox vaccines or Orthopoxvirus vector vaccines and as a vaccine for protective immunization against a smallpox infection.

These objects are solved by the subject-matters of the independent claims. Preferred embodiments are set forth in the dependent claims.

Herein, the identification of immunodominant human leukocyte antigen A*0201-restricted epitopes which are recognized by cytotoxic CD8+ T cells and well conserved among the *Orthopoxviruses,* including variola virus, is reported for the first time. This has permitted an analysis of epitope-specific T cell responses following vaccination of transgenic mice and allowed for a correlation of the immunogenicity of several vaccinia viruses, including the highly attenuated, replication-deficient modified vaccinia virus Ankara (MVA), with protective efficacy against lethal respiratory poxvirus challenge. The data provide a new basis to rationally estimate the efficacy of safe second generation poxvirus vaccines and suggest that such vaccines may already be in hand.

According to a first aspect, the present invention provides an immunogenic Orthopoxvirus antigen comprising at least one of the peptides of SEQ ID NO: 1-50 or variants thereof, wherein said variants comprise one or more insertions, substitutions and/or deletions as compared to the respective peptide of SEQ ID NO: 1-50, wherein the immunogenic activity of the variant is substantially equal to the activity of the unmodified peptide of SEQ ID NO: 1-50. SEQ ID NO: 18 represents a particularly preferred antigen, which has proven an excellent immunogenic activity in mammals. The peptide of SEQ ID NO: 18 is also designated as "VP35#1" in the following. In two different experiments, VP35#1 induced T cell responses which were 37 and 68 times higher than that of a control peptide (hTyr). Further preferred peptides of the present invention, which have induced outstanding immune reponses are SEQ ID NO: 7, 10, 11, 13, 15, 23, 24, 28 and 36.

The term immunogenic activity as used herein is related to the immunogenic function of the inventive peptide sequences to evoke immune responses, in particular immune responses mediated by CD8+ cytotoxic T cells.

The peptide sequences of the present invention also encompass all sequences differing from the herein disclosed sequences by amino acid additions, insertions, deletions, and substitutions.

Preferably, amino acid "substitutions" are the result of replacing one amino acid with another amino acid having similar structural and/or chemical properties, i.e., conservative amino acid replacements. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

"Insertions" or "deletions" are typically in the range of about 1, 2 or 3 amino acids. The variation allowed may be experimentally determined by systematically making insertions, deletions, or substitutions of amino acids in a polypeptide molecule using recombinant DNA techniques and assaying the resulting recombinant variants for activity. This does not require more than routine experiments for the skilled artisan. The terms insertions or deletions as used herein encompass insertions or deletions, which occur adjacent in the respective peptide of the invention or alternatively, which occur separated by one or more of the amino acides of the unmodified peptide of the invention. For example, in a peptide of the invention comprising 10 amino acids, the amino acids in positions 3, 4 and 5 or in positions 2, 4 and 7 may be deleted. Accordingly, also insertions can be made in the indicated or other positions.

The peptides of the present invention may contain one or more epitopes or may form a part of an epitope. The peptides of the present invention preferably contain at least 8-10, more preferred at least 9 amino acids.

Amino acid additions typically are not more than 100, preferably not more than 80, more preferably not more than 50, most preferred not more than 20 amino acids, which are added to the peptides of the present invention at their N- and C-terminus, respectively. It is noted that only those additions are contemplated in this invention, which do not negatively affect the immunogenic activity of the peptides of the present invention, i.e. in particular the HLA-A*0201 restricted cellular immunogenicity of the peptides disclosed herein.

Basically, it is preferred in this application that the variants of the inventive amino acids show at least 50%, more preferably 70 %, most preferably 80% sequence identity to the peptides of SEQ ID NO: 1-50.

Furthermore, the present invention also comprises peptides according to the invention as explained above, which are glycosylated, for example O-glycosylated. This glycosylation may, e.g., occur at one or more of the threonines or serines contained in the sequence. Glycosylated peptides according to this invention may further promote the immune response, therefore leading to an enhanced immune protection against smallpox infections.

The invention further provides an isolated nucleic acid, which codes for one or more of the above described peptides as well as an isolated nucleic acid, which is a transcriptional product of one of the above nucleic acids or, which selectively hybridizes to the above nucleic acids under moderately stringent conditions. The present invention further provides immunogenic antigens, which are encoded by these nucleic acids. The term "nucleic acid sequence" refers to a heteropolymer of nucleotides or the sequence of these nucleotides. The terms "nucleic acid" and "polynucleotide" are used interchangeably herein to refer to a heteropolymer of nucleotides.

Stringency of hybridization, as used herein, refers to conditions under which polynucleotide duplexes are stable. As known to those of skill in the art, the stability of duplex is a function of sodium ion concentration and temperature (see, for example, Sambrook et al., Molecular Cloning: A Laboratory Manual 2^{nd} Ed. (Cold Spring Harbor Laboratory, (1989)). Stringency levels used to hybridize can be readily varied by those of skill in the art.

Low stringency hybridization refers to conditions equivalent to hybridization in 10% formamide, 5 x Denhart's solution, 6 x SSPE, 0.2% SDS at 42°C, followed by washing in 1 x SSPE, 0.2% SDS, at 50°C Denhart's solution and SSPE are well known to those of skill in the art as are other suitable hybridization buffers.

Moderately stringent hybridization refers to conditions that permit DNA to bind a complementary nucleic acid that has about 60% identity, preferably about 75% identity, more preferably about 85% identity to the DNA; with greater than about 90% identity to said DNA being especially preferred. Preferably, moderately stringent conditions are conditions equivalent to hybridization in 50% formamide, 5 x Denhart's solution, 5 x SSPE, 0.2% SDS at 42°C, followed by washing in 0.2 x SSPE, 0.2% SDS, at 65°C.

High stringency hybridization refers to conditions that permit hybridization of only those nucleic acid sequences that form stable duplex in 0.018M NaCl at 65°C. (i.e., if a duplex is not stable in 0.018M NaCl at 65 °C, it will not be stable under high stringency conditions, as contemplated herein).

Further, nucleic acid hybridization techniques can be used to identify and obtain a nucleic acid within the scope of the invention. Briefly, any nucleic acid having some homology to a sequence set forth in this invention, or fragment thereof, can be used as a probe to identify a similar nucleic acid by hybridization under conditions of moderate to high stringency. Such similar nucleic acid then can be isolated, sequenced, and analyzed to determine whether they are within the scope of the invention as described herein. Furthermore, nucleic acids are contemplated in this invention, which comprise nucleic acid changes due to the degeneracy of the genetic code, which code for the same or a functionally equivalent peptide in accordance with SEQ ID NO: 1-50.

Generally, the term "nucleic acid" as used herein encompasses both RNA and DNA, including cDNA, genomic DNA, and synthetic (e. g., chemically synthesized) DNA. The nucleic acid can be double-stranded or single-stranded. Where single-stranded, the nucleic acid can be the sense strand or the antisense strand. In addition, nucleic acid can be circular or linear.

The term "isolated" as used herein with reference to nucleic acid refers to a naturally-occurring nucleic acid that is not immediately contiguous with both of the sequences with which it is immediately contiguous (one on the 5'end and one on the 3'end) in the naturally-occurring genome of the organism from which it is derived.

For example, an isolated nucleic acid can be, without limitation, a recombinant DNA molecule of any length, provided one of the nucleic acid sequences normally found immediately flanking that recombinant DNA molecule in a naturally-occurring genome is removed or absent. Thus, an isolated nucleic acid includes, without limitation, a recombinant DNA that exists as a separate molecule (e. g., a cDNA or a genomic DNA fragment produced by PCR or restriction endonuclease treatment) independent of other sequences as well as recombinant DNA that is incorporated into a vector, an autonomously replicating plasmid, a virus (e. g., a retrovirus, adenovirus, or herpes virus), or into the genomic DNA of a prokaryote or eukaryote. In addition, an isolated nucleic acid can include a recombinant DNA molecule that is part of a hybrid or fusion nucleic acid sequence.

The term "isolated" also includes any non-naturally-occurring nucleic acid since non-naturally-occurring nucleic acid sequences are not found in nature and do not have immediately contiguous sequences in a naturally-occurring genome. For example, a non-naturally-occurring nucleic acid such as an engineered nucleic acid is considered to be an isolated nucleic acid. Engineered nucleic acid can be made using common molecular cloning or chemical nucleic acid synthesis techniques. Isolated non-naturally-occurring nucleic acids can be independent of other sequences, or incorporated into a vector, an autonomously replicating plasmid, a virus (e. g., a retrovirus, adenovirus, or herpes virus), or the genomic DNA of a prokaryote or eukaryote. In addition, a non-naturally-occurring nucleic acid can include a nucleic acid molecule that is part of a hybrid or fusion nucleic acid sequence.

According to a further aspect, the present invention provides a recombinant vector, which comprises one or more of the above described nucleic acid sequences.

According to a preferred embodiment, the nucleic acids according to the invention are provided in an expression vector. This expression vector preferably comprises one or more regulatory sequences. The term "expression vector" generally refers to a plasmid or phage or virus or vector, for expressing a polypeptide from a DNA (RNA) sequence. An expression vector can comprise a transcriptional unit comprising an assembly of (1) a genetic element or elements having a regulatory role in gene expression, for example, promoters or enhancers, (2) a structural or coding sequence which is transcribed into mRNA and translated into protein, and (3) appropriate transcription initiation and termination sequences. Structural units intended for use in yeast or eukaryotic expression systems preferably include a leader sequence enabling extracellular secretion of translated protein by a host cell. Alternatively, where recombinant protein is expressed without a leader or transport sequence, it may include an N-terminal methionine residue. This residue may or may not be subsequently cleaved from the expressed recombinant protein to provide a final product.

The present invention further provides hosts, e.g. host cells, which have been transformed to contain the polynucleotides of the invention. The term "transformation" means introducing DNA into a suitable host cell so that the DNA is replicable, either as an extrachromosomal element, or by chromosomal integration.

For example, such host cells may contain nucleic acids of the invention introduced into the host cell using known transformation methods. The present invention still further provides host cells genetically engineered to express the polynucleotides of the invention, wherein such polynucleotides are in operative association with a regulatory sequence heterologous to the host cell which drives expression of the polynucleotides in the cell.

The host cell can be a higher eukaryotic host cell, such as a plant cell, a mammalian cell, a lower eukaryotic host cell, such as a yeast cell, or can be an insect cell, or the host cell can be a prokaryotic cell, such as a bacterial cell. Introduction of the recombinant construct into the host cell can be effected by calcium phosphate transfection, DEAE, dextran mediated transfection, or electroporation (Davis, L. et al., Basic Methods in Molecular Biology (1986)).

The most preferred cells are those which do not normally express the particular polypeptide or protein or which express the polypeptide or protein at low natural level. Mature proteins can be expressed in mammalian cells, yeast, bacteria, or other cells under the control of appropriate promoters. Appropriate cloning and expression vectors for use with prokaryotic and eukaryotic hosts and the corresponding methods are described by Sambrook, et al., in Molecular Cloning: A Laboratory Manual, 2^{nd} Edition, Cold Spring Harbor, New York (1989).

The mammalian cell is pereferably a CHO, COS, HeLa, 293T, HEH or BHK cell.

Bacterial cells comprise Streptococci, Staphylococci, E. coli, Streptomyces and Bacillus subtilis cells. *E.coli* and *Bacillus subtilis* are preferred. Also fungal cells can be used, such as yeast cells (from *Saccharomyces*, *Schizosaccharomyces*, *Pichia)* and Aspergillus cells. As insect cells, Drosophila S2 and Spodoptera Sf9 cells are preferred. As plant cells, cells from *N. tabacum* or *Arabidopsis thaliana* may be used.

According to a further aspect of the present invention, antigen presenting cells are provided which have been pulsed with one or more of the peptides of the present invention. Preferably, these antigen presenting cells are dendritic cells or B cells.

Furthermore, the present invention provides a diagnostic composition comprising one or more of the inventive peptides or the aforementioned antigen presenting cells. For explanations how to use those peptides and antigen presenting cells (APC's) in a diagnostic method, see below.

Additionally, a diagnostic kit for detecting an immune response against smallpox vaccines or Orthopoxvirus vector vaccines is provided comprising at least one or more of the inventive peptides and/or the inventive APC.

According to a further aspect, a pharmaceutical composition is provided comprising a therapeutically effective amount of one or more peptides of the invention or one or more of the inventive nucleic acids, and a pharmaceutically acceptable carrier.

Furthermore, the present invention provides a pharmaceutical composition, comprising a therapeutically effective amount of one or more peptides of the invention, one or more of the nucleic acids or a vector of the invention, and a pharmaceutically acceptable carrier. Such a composition may also contain (in addition to the above identified ingredients and the carrier) diluents, fillers, salts, buffers, stabilizers, solubilizers and other materials well known in the art. The term "pharmaceutically acceptable" means a non-toxic material that does not interfere with the effectiveness of the biological activity of the active ingredient(s). The characteristics of the carrier will depend on the route of administration. The therapeutic composition may further contain other agents which either enhance the activity or use in treatment. Such additional factors and/or agents may be included in the therapeutic composition to produce a synergistic effect or to minimize side-effects.

Techniques for formulation and administration of the compounds of the present application may be found in "Remington's Pharmaceutical Sciences", Mack Publishing Co., Easton, PA, latest edition.

The compositions contain a therapeutically effective dose of the respective ingredient. A therapeutically effective dose refers to that amount of the compound sufficient to result in amelioration of symptoms, e.g., treatment, healing, prevention or amelioration of such conditions, specifically in an induction of an immune response in the patient treated. Suitable routes of administration may, for example, include parenteral delivery, including intramuscular and subcutaneous injections, as well as intrathecal, direct intraventricular, intravenous, intraperitoneal injections. Intravenous administration to the patient is preferred.

A typical composition for intravenous infusion can be made up to contain 250 ml of sterile Ringer's solution, and 10 mg of the ingredient. See Remington's Pharmaceutical Science (15^{th} Ed., Mack Publishing Company, Easton, Ps., 1980). Preferably, the therapeutic composition of the present invention is a vaccine.

As mentioned above, this vaccine finds application for use in the protective immunization against a smallpox infection.

According to a further aspect, the present invention is directed to an ex vivo-method of detecting a T cell response induced by smallpox vaccines or Orthopoxvirus vector vaccines comprising the following steps:
a) providing a sample containing T cells from a subject, which subject has been immunized with a smallpox vaccine or Orthopoxvirus vector vaccine,
b) co-cultivating said sample with one or more of the peptides of the present invention or a diagnostic composition as disclosed herein, under conditions, which allow an activation of said T cells, and
c) determining the activity of T cells contained in that sample specific against any amino acid indicated in b).

As used herein, smallpox vaccines are vaccines on the basis Orthopoxvirus antigens suitable for vaccination of human individuals, preferably these are live Orthopoxvirus vaccines, preferably based on vaccinia virus. Vaccinia viruses suitable for use as a smallpox vaccine include known vaccinia virus vaccine strains, e.g. New York City Board of Health, Elstree or Lister, or Copenhagen strains, and attenuated vaccinia viruses including Modified vaccinia virus Ankara (MVA), NYVAC, LC16m8, defective viruses habouring deletions of essential gene sequences (e.g. D4R), or viruses with modulated virulence or immunogenicity due to containing specific mutations and/or deletions in non-essential gene sequences (e.g. thymidine kinase gene, VGF gene, or E3L gene sequences) or carrying recombinant gene sequences (e.g. IL-2 or IL-15 gene sequences).

Orthopoxvirus vector vaccines are based on recombinant Orthopoxviruses that express heterologous gene sequences of pathogens and have the capacity to deliver pathogen antigens to induce or stimulate immune responses. Recombinant Orthopoxviruses are preferably recombinant vaccinia viruses including attenuated vaccinia viruses including Modified vaccinia virus Ankara (MVA), NYVAC, LC16m8, defective viruses habouring deletions of essential gene sequences (e.g. D4R), or viruses with modulated virulence or immunogenicity due to containing specific mutations and/or deletions in non-essential gene sequences (e.g. thymidine kinase gene, VGF gene, or E3L gene sequences) or carrying recombinant gene sequences (e.g. IL-2 or IL-15 gene sequences). Preferred pathogens include viruses (e.g. HIV-1, HCV), bacteria (e.g. mycobacterium tuberculosis), parasites (Plasmodium falciparum), and tumors (e.g. melanoma).

The term activation, as used herein, is defined as a stimulation of T cells. The cocultivation step performed in b) is necessary in order to activate T cells contained in the sample. This can be performed by either directly applying the amino acids of the present invention to the sample, preferably the blood sample, obtained from the patient. Thus, peripheral blood mononuclear cells (PBMC), which contain the T cells, and which can be easily prepared from patient blood samples, provide APC's (e.g. B cells), which thereby are pulsed by the inventive peptides.

Alternatively, those pulsed APC's can be separately prepared before they are used in step b), simply by pulsing APC's, preferably autologous APC's, with the inventive peptides in accordance with methods well known in the art and then co-cultivating the sample with said APC's. These APC's are preferably so selected that they express the appropriate MHC class I molecule.

The T cells, the activity of which can be determined by the inventive method preferably are cytotoxic T cells, more preferably CD 8+ T cells.

The activity of the T cells in the above step c) can be determined by methods, which are per se known in the art. Further information can be found in, for example, *Immunology*, *5*^{*th*} *edition, 2001*.

According to one embodiment, this determination can be performed by means of a ⁵¹Cr-release assay. Specifically, the T-cell function can be determined by a ⁵¹Cr-release assay using a T-cell bioassay, which is based on the killing of a target cell by a cytotoxic T cell. This assay is based on the uptake of radioactively labeled sodium chromate, Na₂⁵¹CrO₄, by living cells, which do not again spontaneously release that sodium chromate. When these labeled cells are killed, the radioactive chromate is released and its presence in the supernatant of mixtures of target cells and cytotoxic T cells can be measured. An example of target cells are T2 cells, which have been pulsed with one or more of the inventive amino acids.

According to a preferred embodiment, the determination in step c) is performed by measuring the amount of cytokines in said T cells. For this purpose, the following methods can be employed:

At first, the cytokines can be measured by intracellular cytokine staining.

The approach of intracellular cytokine staining relies on the use of metabolic poisons that inhibit protein export from the cell. Then, the cytokine accumulates within the endoplasmic reticulum and vesicular network of the cell. If the cells are subsequently fixed and rendered permeable by the use of mild detergents, antibodies can gain access to these intracellular compartments and are able to detect the cytokine.

Alternatively, the cytokines are measured extracellular by an ELISPOT assay. The ELISPOT assay is a modification of an ELISA antigen-capture assay. It is an excellent approach for measuring the frequency of T-cell responses. Populations of T cells are stimulated with the antigen of interest, and are then allowed to settle onto a plastic plate coated with antibodies which are directed against the cytokine that is to be assayed. All cytokine secreted by the T cell is captured by the antibody on that plastic plate. After a defined time period the cells are removed, and a second antibody to the cytokine is added to the plate to reveal a circle of bound cytokine surrounding the position of each activated T cell, counting each spot, and knowing the number of T cells originally added to the plate allows a simple calculation of the frequency of T cells secreting that particular cytokine.

The sample, which contains the T cells, which are to be analysed, is preferably a body fluid, more preferably a blood sample of a mammal, preferably a human. Thus, the sample can be easily obtained from a patient and thus, the degree of immune protection against a smallpox infection following a vaccination can be easily determined.

For example, the inventive method can be performed as follows: peripheral blood mononuclear cells (PBMC), which contain the T cells, can be easily prepared from patient blood samples. The virus antigen-specific T cells within the PBMC preparation (i) can be analysed directly *ex* vivo or (ii) can be further amplified in in vitro cultures for further, e.g. functional, testing. The latter typically includes the measurement of bulk cytotoxicity mediated by CD8+ T cells that have been in vitro stimulated and amplified in co-culture experiments using (e.g. peptide-specific) antigens and autologous antigen-presenting cells (APC) for several days/weeks. For ex *vivo* analysis the antigen-specific T cells of a PBMC preparation are typically short-term stimulated with peptides and/or APC for several hours before being tested, e.g. using ELISPOT to measure IFN-γ spot forming cells, or MHC class I-peptide tetramer assay or intracellular cytokine detection (e.g. IFN-γ) assay to quantify and characterize antigen-specific T cells by flow cytometry.

Unless otherwise defined, all technical and scientific terms used herein have the same meaning as commonly understood by one of ordinary skill in the art to which this invention pertains. All publications, patent applications, patents, and other references mentioned herein are incorporated by reference in their entirety. In case of conflict, the present specification, including definitions, will control. In addition, the materials, methods, and examples are illustrative only and not intended to be limiting.

The invention is now further illustrated by Examples and the accompanying drawings, which are showing the following:

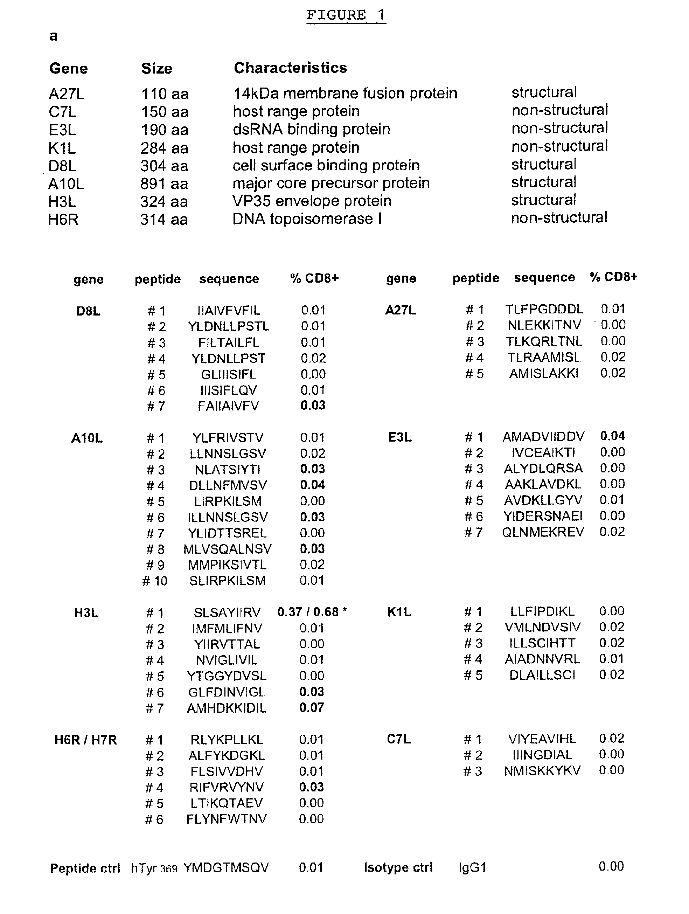

Fig. 1. HLA-A*0201-restricted peptide-epitopes derived from vaccinia virus-encoded proteins. (*a*) Peptide-specific HLA-A*0201-restricted IFN_{γ} secretion of splenocytes derived from vaccinia virus-immunized HHD mice. Mice were vaccinated intraperitoneally with 10⁸ IU MVA. Ten days post infection, spleens were harvested, splenocytes pooled and fractions incubated with the above indicated A*0201-binding peptides. Splenocytes were analysed for the presence of peptide-specific CD8+ cells. The magnitude of the respective T cell response is depicted as percentages of cytokine secreting CD8+ cells within the living CD8+ T cell population (% CD8+). Peptide-specific responses being higher than double the background response against an irrelevant HLA-A*0201-binding peptide (huTyr 369) used as a negative control were calculated as positive and are written in bold letters. The numbers marked by an asterisk refer to results from two independent experiments. The D8L peptides correspond to SEQ ID NO: 1-7, the A10L peptides to SEQ ID NO: 8-17 etc. (*b*) Alignment of VP35#1-peptide sequences derived from poxvirus gene products homologous to the vaccinia virus H3L gene encoded protein: Vaccinia virus strains Modified vaccinia virus Ankara (VV-MVA; AAB96447), Copenhagen (VV-Cop; AAA48090), Western Reserve (VV-WR; AAB59838) and Tian Tan (VV-Tan; AAF33961); monkeypox virus strain Zaire-96-I-16 (MPXV-ZRE; AAL40551); camelpox virus isolate M-96 (CPXV-K/M96; NP_570489); ectromelia virus (EV; www.sanger.ac.uk); variola major virus strains Bangladesh-1975 (VAR-BSH; AAA60834) and India-1967 (VAR-IND; CAA49027); variola minor virus strain Garcia-1966 (VMN-GAR; CAB54686); yaba-like disease virus (YAB-YLD; CAC21312); molluscum contagiosum virus subtype 1 (MC1; AAC55212); fowlpox virus (FPV-FCV; AAF44484); orf virus strains NZ2 (Orf-NZ2; AF097215), OV/20 (Orf-OV/20; AY040085) and OV/7 (Orf-OV/7; AY040084); myxoma virus strain Lausanne (MYX-LAU; AAF14959); lumpy skin disease virus isolate Neethling 2490 (LSD-NEE; AAK85035); swinepox virus isolate 17077-99 (SPV-KAS; AF410153).

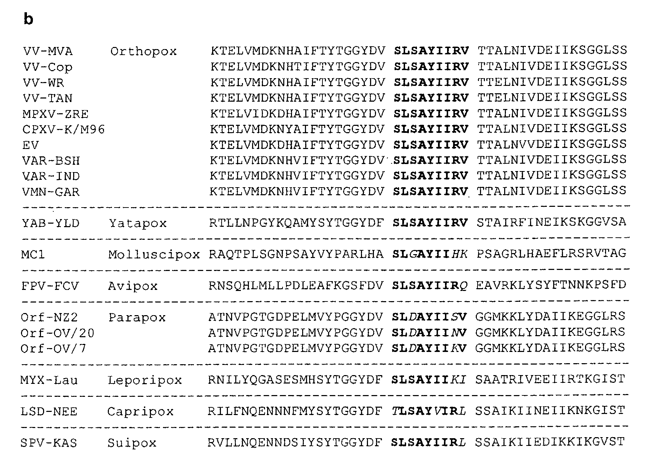

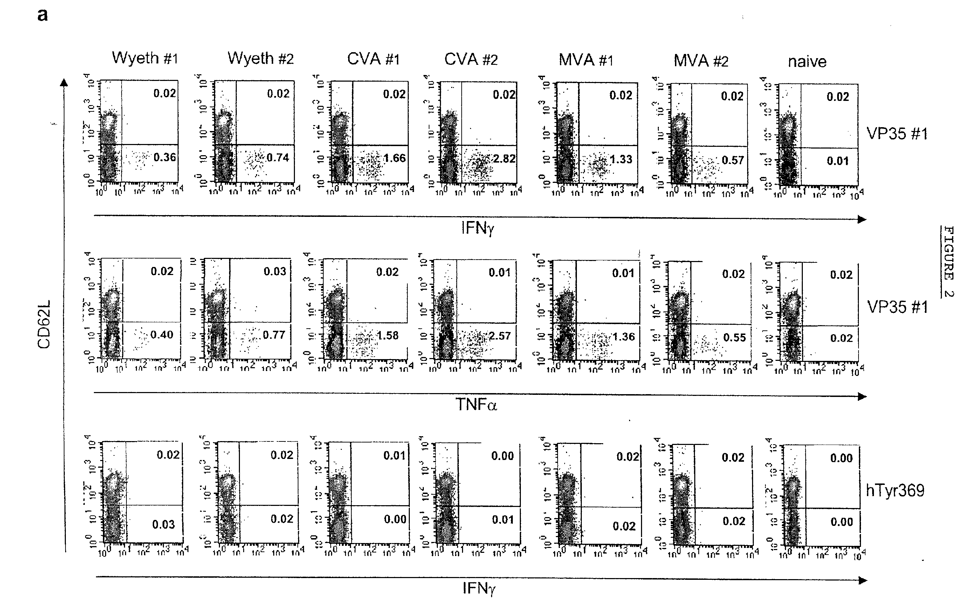

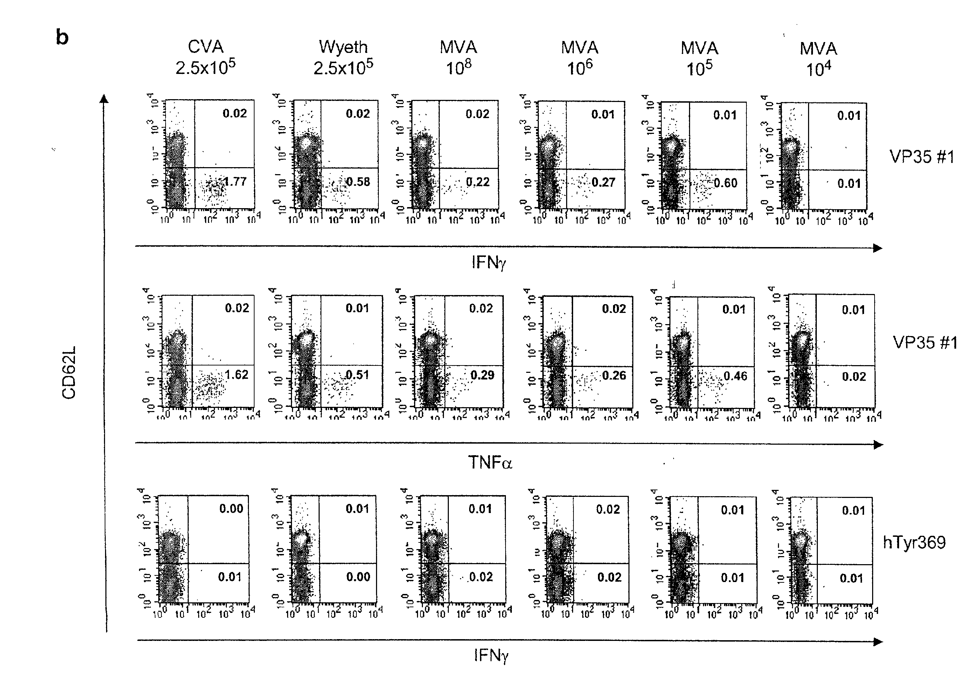

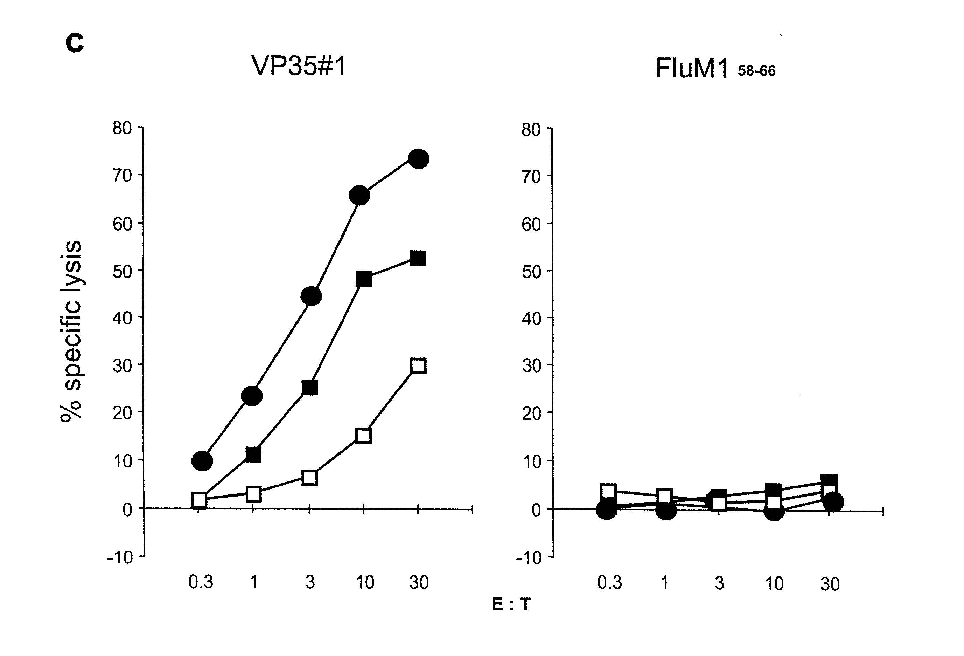

Fig. 2. HLA-A*0201-restricted cellular immunogenicity of various smallpox vaccines. *(a* and *b)* Peptide-specific intracellular cytokine release of splenocytes derived from vaccinated HHD mice. Mice were immunized i.p. (*a*) with 2.5 x 10⁵ PFU of replication-competent vaccinia virus CVA or Wyeth, or 10⁸ IU of replication-deficient vaccinia virus MVA; #1 and #2 refer to individual mice analysed; i.m. (*b*) with various doses of virus: 2.5 x 10⁵ PFU of CVA or Wyeth, or 10⁸ IU, 10⁶ IU, 10⁵ IU and 10⁴ IU for MVA. Flow cytometric analysis was performed with cells stained with EMA, PE-anti-CD8, APC-anti-CD62L and FITC-anti-IFNγ or -anti-TNFα or the respective FITC-labelled isotype control. Splenocyte preparations were analysed for the presence of VP35#1 peptide-specific, activated (CD62L) CD8+ cells. The magnitude of the induced T cell response is depicted as percentages of cytokine secreting CD8+ cells within the live (EMA⁻) and CD8+ cell population. (*c*) Specific lysis of peptide-pulsed human target cells by VP35#1 peptide-specific CTL derived from vaccinated HHD mice. Effector CTLs were generated by single i.p. immunization of Tg mice with either MVA (■), CVA (•), or Wyeth (□). CTLs were assayed for cytotoxicity at the indicated E:T ratio in a 4-h ⁵¹Cr release assay against T2 targets pulsed with either the VP35#1 peptide or the influenza M1 58-66 peptide at 1 µM.

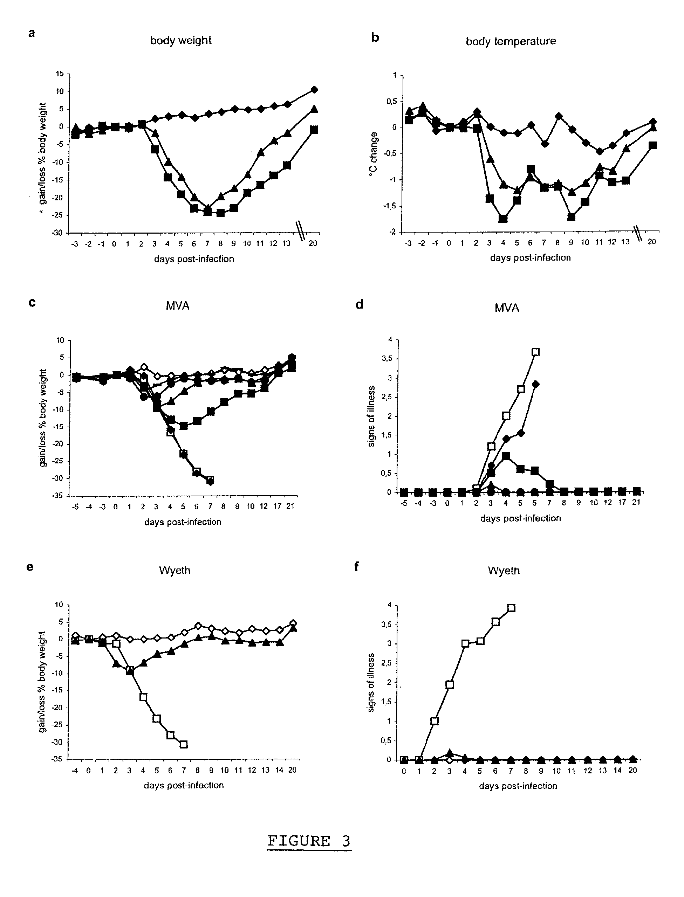

Fig. 3. Analysis of virus virulence and vaccine-induced protection in a mouse model for respiratory poxvirus infection. *(a* and *b)* Characterization of infections with MVA, CVA, and WR. Mice (n=8-10) were inoculated by the intranasal route with 1x10⁸ IU MVA [◆], 5x10⁵ PFU CVA [▲] and 3x10⁴ PFU WR [■]. Body weight (*a*) and body temperature (*b*) was monitored daily and is expressed as mean for each group. (c *- f)* Protective efficacy of vaccination. Mice (n=10) were immunized intramuscularly with 10⁴ [◆], 10⁵ [■], 10⁶ [A], 10⁷ [•], 10⁸ [-] IU of MVA (C, D) or 10⁵ [▲] PFU of Wyeth (*e* and *f*). Three weeks after vaccination animals were challenged intranasally with 1 x 10⁶ PFU WR. Individual animal weights (*c* and *e*) and signs of illness (*d* and *f*) were monitored daily and are expressed as means for each group. Mock vaccinated [□] and mock challenged [0] mice served as control groups.

### Methods

Viruses. Vaccinia virus strains Wyeth-New York City Board of Health and Western Reserve were originally provided by Bernard Moss (NIH, Bethesda, USA), vaccinia virus strains CVA and MVA were originally obtained from Anton Mayr (University of Munich, Germany), and CVA from the 2^{nd} and MVA from the 582^{nd} (cloned isolate F6) passage on CEF were used for this study. All viruses were propagated and titered following standard methodology (10). To generate vaccine preparations, viruses were routinely purified by ultracentrifugation through sucrose and reconstituted in 10 mM Tris pH 7.4, 120 mM NaCl saline buffer.

Screening for HLA-A*0201-restricted peptides. HLA-A*0201-restricted peptide-epitopes derived from vaccinia virus-encoded proteins were selected by epitope prediction using the SYFPEITHI database (11). The converted internal tyrosinase 369-377 peptide YMDGTMSQV and the influenza virus A/PR/8/34 matrix protein M1 58-66 peptide GILGFVFTL both of which were known to bind to HLA-A*0201 (12) were used as negative controls. Purity of peptides was ascertained by mass spectometry and HPLC to be >90%.

Animal models. Female six to eight week-old transgenic HHD^{+/+} ß₂m^{-/-} D^{b-/-} mice (HHD) (13) were used for immunization experiments inoculating 0.5 or 0.1 ml volumes of virus vaccine by the intraperitoneal or intramuscular route, and monitoring for HLA-A*0201-restricted T cell responses at days 10 to 28 after vaccination. For protection assays, groups of female BALB/c mice (n=8 to 10) were intramuscularly vaccinated once with 0.1 ml virus vaccine. Three weeks after immunization, animals were anesthesized, infected intranasally with vaccinia virus Western Reserve diluted in 30 µl phosphate buffered saline, and monitored for at least further three weeks with daily measurement of individual body weights and scoring of signs of illness as described previously (14). Animals suffering from severe systemic infection and having lost >30% of body weight were sacrificed. The mean change in body weight was calculated as the percentage of the mean weight for each group on the day of challenge. Body temperature was determined with a Electronic Laboratory Animal Monitoring System (BioMedic Data Systems, Marywood, NJ) using subcutaneously implanted microchip battery-free transponders and a DAS-5004 Pocket Scanner for data collection. Mean changes in body temperatures were calculated by substracting the pre-challenge (days -3 to 0) baseline temperature of each group from each subsequent time point.

Cytokine staining assays. Ten days after vaccination, splenocytes from vaccinia virus-immunized HHD mice were prepared and incubated for 5 h with A*0201-binding peptides at 10⁻⁶ M. After two hours, Brefeldin A was added at a final concentration of 1 µg/ml (GolgiPlug^{TM}; PharMingen Becton Dickinson). Cells were live/dead stained in PBS/1%BSA/1µg/ml ethidium monoazide bromide (EMA; Molecular Probes) and blocked for unspecific FcγIII and -II receptor-mediated binding with 5µg/ml purified anti-CD16/CD32 (Fc Block^{TM}; PharMingen Becton Dickinson) for 20 min at 4°C. After light inactivation of EMA, cell surface staining was performed with PE-anti-CD8 (53-6.7) and APC-anti-CD62L (Mel-14) for 30 min at 4°C. Subsequently, intracellular cytokine staining was performed for 30 min at 4°C applying FITC-anti-IFNγ (XMG1.2) or FITC-anti-TNFα (MP6-XT22) or the respective FITC-labelled IgG1 isotype control (R3-34) using Cytofix/Cytoperm^{TM} Kit (all PharMingen Becton Dickinson). Splenocytes were analysed by flow cytometry (FACScalibur) using CellQest® software (both Becton Dickinson).

Chromium release assays. Ten days after the vaccination, spleen cells of primed mice were co-cultured with irradiated (3000 rad) and LPS-activated syngeneic spleen cell stimulators which had been pulsed with VP35#1 or the tyrosinase 369-377 control peptide at 5 µg/ml and human ß₂-microglobulin (Sigma Chemical Co., St. Louis, MO) at 10 µg/ml. After 6 days of culture, the lytic activity of resultant CTL effector cells was determined at various E/T ratios in a 4-h standard ⁵¹Cr-release assay (12). As targets served T2 cells which had been pulsed at 1 µM with VP35#1 peptide. The tyrosinase 369-377 peptide or the influenza M1 58-66 peptide served as control peptides for pulsing of stimulator or target cells.

### Results

The genome of vaccinia virus, the most representative member of the *Orthopoxvirus* genus, encodes for more than 200 viral proteins that may serve as possible antigens (15). In our attempt to identify immune correlates of successful poxvirus-specific immunization, we concentrated on vaccinia virus gene products that (i) share high amino acid homologies to variola virus antigens (16), (ii) were known to be immunodominant with regard to induction of virus-specific antibodies (17, 18), or (iii) are non-structural virus proteins being produced during early viral gene expression and possibly preferentially suitable for MHC class I-associated immune presentation (19). Because of the dominant representation of HLA-A*02 in human populations, we sought to identify poxvirus polypeptides allowing for HLA-A*0201-specific restriction of peptide epitopes. Using epitope prediction as offered by the SYFPEITHI database of MHC ligands and peptides (11) we preselected eight vaccinia virus polypeptide sequences (Fig. 1*a*) and defined peptides with highest probability for association with HLA-A*0201. These peptides were then tested for their ability to specifically induce cytokine production in stimulated splenocytes from vaccinia virus-infected HLA-A*0201-transgenic mice (HHD) (13). Monitoring T cells by FACS analysis after IFNγ-specific intracellular staining, we could identify multiple peptides which induced IFN_{γ} release above background levels, and including the peptide VP35#1 which had particular stimulating capacity for up to 0.7% of all CD8+ T cells (Fig 1*a*). VP35#1 is part of a 35 kDa vaccinia virus envelope protein being encoded by the ORF H3L and described as an immunodominant antigen (18, 20). Comparison of the VP35#1 sequence with known H3L gene homologues derived from various poxviruses revealed complete conservation of the peptide among *Orthopoxviruses* including variola major and variola minor. Interestingly, VP35#1 is also conserved in the more heterologous protein sequence from Yaba-like disease virus, a member of the *Yatapoxvirus* genus which can cause infections in humans (21), whereas amino acid substitutions occur in sequences of poxviruses from other genera (Fig. 1*b*).

To comparatively characterize VP35#1-specific T cell responses induced after immunization, we vaccinated HHD mice with the different vaccinia virus strains New York City Board of Health (Wyeth), chorioallantois vaccinia virus Ankara (CVA) or modified vaccinia virus Ankara (MVA), all of them being previously used as smallpox vaccines. Wyeth and CVA are replication-competent viruses which were grown on live calves or primary bovine tissue culture for production of smallpox vaccine, and used for multipuncture intradermal vaccination in humans (5, 22). MVA was originally developed from CVA by serial passage in chicken embryo fibroblast cultures (CEF) and tested in Germany as safe tissue culture prepared vaccine (23). MVA is severely host range restricted, serves as vector virus for recombinant gene expression, and as candidate vaccine for delivery of heterologous antigens (24, 25).

Relying on our previous experience using MVA vector vaccines in HLA-A*0201-transgenic mice we immunized animals with 10⁸ infectious units (IU) MVA by the intraperitoneal (i.p.) route (12). In contrast, vaccines based on replication competent viruses CVA and Wyeth were given i.p. at a dose of 2.5x10⁵ plaque forming units (PFU), corresponding to an infectious dose of CVA that when previously used in mice resulted occasionally in generalizing infections but not in mortality (26). A single inoculation of all vaccinia viruses was able to induce VP35#1-specific activated CD8+ T cells as revealed by FACS analysis of freshly prepared splenocytes after intracellular staining for IFNγ and TNFα (Fig 2*a*). Highest levels of VP35#1-reactive T cells secreting IFNγ as well as TNFα were detected after vaccination with CVA, whereas immunizations with Wyeth or MVA elicited comparable responses e.g. ranging from 0.36 - 1.33 % IFN_{γ} releasing T lymphocytes of all splenic CD8+ T cells. To assess the vaccines in a more realistic scenario for vaccination, we analysed immune responses in HHD mice inoculated once by the intramuscular route. Additionally, we wished to monitor for possible effects of MVA replication deficiency, and included animals that had received decreasing doses of MVA vaccine. Despite the less "systemic" nature of this vaccination route we found similar levels VP35#1-specific T cell responses as observed after intraperitoneal injection (Fig. 2*b*). Surprisingly, vaccination with MVA using doses from 10⁵ to 10⁸ IU elicited comparable immune responses, while we failed to detect VP35#1-specific T cells after inoculation of MVA vaccines containing less infectivity (Fig. 2*b*, 10⁴ IU). To test the peptide-reactive T cells also for their cytotoxic capacities, we assayed splenocytes from HHD mice vaccinated with MVA, CVA, or Wyeth against VP35#1-specific human target cells (Fig. 2*c*). Efficient specific lysis was found with effector cells from all vaccinees. Again, highest levels of cytotoxicity were obtained after vaccination with CVA, closely followed by CTL responses induced with MVA and Wyeth.

Evaluating the efficacy of vaccines with regard to protective capacities is another important issue for the development of new *Orthopoxvirus*-specific vaccines. To assess how our data regarding vaccine immunogenicities would compare to vaccine protection, we took advantage of the fact that respiratory infection of mice represents a well established animal model to monitor virulence of vaccinia viruses including virus mutants (27, 14). Monitoring the infection of BALB/c mice with CVA or vaccinia virus Western Reserve (WR), a laboratory strain also well characterized with regard to its virulence in mice, we observed the onset of a typical disease pattern that peaked at about seven days after infection and which was accompanied by the loss of body weight (Fig. 3a), reduced ability to maintain body temperature (Fig. 3*b*), and typical signs of illness including disturbed fur, arched back, and respiratory symptoms. Corresponding to previously established lethal doses 50 (LD50) for WR (27), inoculation of 3 x 10⁴ PFU WR resulted in death of two out of eight animals, whereas all but one of the animals survived the infection with 5 x 10⁵ PFU CVA. In contrast, inoculations of 10⁸ IU MVA were tolerated without any signs of disease becoming apparent, a result which also in this model system confirms the avirulent phenotype of replication-deficient MVA already described in the literature (23). This high degree of attenuation (together with its immunogenicity) makes MVA to an obvious candidate for a second-generation Orthopoxvirus vaccine albeit with a need to evaluate its protective capabilities.

Finally, matching our immunization experiments to determine cytotoxic T cell responses in HHD mice, we then vaccinated BALB/c mice by single intramuscular injection of 10² to 10⁸ IU MVA vaccine. Three weeks after immunization, we submitted the animals to a respiratory infection with 10⁶ PFU WR which corresponds to at least 20 LD50 of this virus strain (27). As shown in Figures 3c and 3d all animals having received 10⁵ IU or higher doses MVA vaccine survived the challenge, whereas NO: protection was seen after inoculation with 10⁴ or less IU MVA. Monitoring of bodyweight and signs of disease suggested that the levels of protection correlated well with increased dosage of the vaccine. NO: signs of illness and only a short transient reduction in body weight was seen in mice immunized with 10⁶ IU MVA. This level of protection closely resembled the efficacy of vaccinations with 10⁵ PFU Wyeth which we used as control vaccine representing a replication-competent vaccinia virus with well established protective capacity (Fig. 3*e*, *3f).*

Here we identified a set of vaccinia virus derived peptides which represent epitopes for HLA-A*0201-restricted CD8+ T cell responses. While immune responses against multiple MHC I and MHC II-restricted viral antigens are likely important for the *in vivo* control of poxvirus infections, these epitopes may allow for a more detailed experimental monitoring of cellular immune responses induced by poxvirus-specific immunization including vaccination of HLA-A*0201-positive humans. The highly immunogenic and well conserved peptide VP35#1 is processed from a virus antigen which was previously described as a target of immunodominant antibody responses (18). Remembering that antibody screening can be used for identification of human tumor antigens being CTL targets (28), further evaluation of virus proteins known to elicit good antibody responses might prove useful for deriving other immunodominant poxvirus-specific T cell epitopes. The identification of the VP35#1 epitope has allowed, for the first time, a direct comparison of the T cell responses induced by live vaccinia viruses in a single antigen-specific manner *ex vivo.* Our data suggest that different vaccinia virus strains may vary to some degree for their ability to elicit epitope-specific T cells, similar to observations made earlier with regard to neutralizing antibodies found after smallpox vaccination (29). We detected peak T cell responses after immunization with CVA, which was described to prompt higher incidence of multiple lesions developing at the site of inoculation when tested as smallpox vaccine in Germany (22). After vaccination with the less virulent but replication-competent strain Wyeth or highly attenuated replication-deficient MVA, we found comparable amounts of VP35#1-specific T cells. This result was reminiscent of the comparable immunogenicities determined for Wyeth or MVA vector vaccines upon vaccination of rhesus macaques against simian immunodeficiency virus (30). The fact that we found quite prominent T cell responses after immunization with either 10⁸, 10⁶, or 10⁵ IU MVA, but failed to detect any specific T cells giving lower doses of MVA vaccine may point towards the requirement for threshold levels of antigen to induce specific T cells. Importantly, our in vivo titrations of MVA demonstrate that inoculation of vaccine doses that induced detectable VP35#1-specific CD8+ T cells also protected against lethal respiratory infection with virulent vaccinia virus Western Reserve. The impact of challenge was most accurately reflected by changes in animal body weights followed by the onset of signs of illness. Furthermore, we show that highly attenuated MVA, despite it's likely incapability to replicate *in vivo*, can provide levels of vaccine protection equal to those induced by the conventional vaccine virus Wyeth, yet with the requirement for about 10-fold higher dosage. The latter may be interpreted as compensation for the *in vivo* replication of Wyeth naturally providing higher levels of antigen upon immunization. While we believe that both CD4+ and CD8+ T lymphocytes are important for in vivo control of *Orthopoxvirus* infections (8), the new knowledge of virus-derived peptide specificities, will allow to carefully evaluate the role of epitope-specific CD8+ T cells in vaccine protection. Taken together, our results presented here suggest that MVA may be a suitable vaccine against smallpox should events call for its wide use in humans, that is safer yet as effective than the current vaccine.

### References

- 1.: Lane, H. C., Montagne, J. L. & Fauci, A. S. (2001) *Nat. Med.* 7, 1271-1273.
- 2.: Gani, R. & Leach, S. (2001) *Nature* 414, 748-751.
- 3.: Fenner, F., Henderson, D. A., Arita, I., Jezek, Z. & Ladnyi, I. (1988) *Smallpox and its eradication* (World Health Organization, Geneva).
- 4.: Birmingham, K. & Kenyon, G. (2001) *Nat. Med.* 7, 1167.
- 5.: Rosenthal, S. R., Merchlinsky, M., Kleppinger, C. & Goldenthal, K. L. (2001) *Emerg. Infect. Dis.* 7, 920-926.
- 6.: Henderson, D. A. & Moss, B. (1999) in *Vaccines*, eds. Plotkin, S. A. & Orenstein, W. A. (W.B. Saunders Company, Philadelphia), Vol. Chapter 6.
- 7.: Freed, E. R., Duma, R. J. & Escobar, M. R. (1972) *Am. J. Med.* 52, 411-420.
- 8.: Karupiah, G., Buller, R. M. L., Vanrooijen, N., Duarte, C. J. & Chen, J. H. (1996) *J. Virol.* 70, 8301-8309.
- 9.: Demkowicz, W. E., Jr., Littaua, R. A., Wang, J. & Ennis, F. A. (1996) *J. Virol.* 70, 2627-2631.
- 10.: Moss, B. & Earl, P. L. (1991) in *Current Protocols in Molecular Biology*, eds. Ausubel, F. M., Brent, R., Kingston, R., Moore, D., Seidman, J., Smith, J. & Struhl, K. (John Wiley & Sons, New York), pp. 16.16.1-16.16.7.
- 11.: Rammensee, H., Bachmann, J., Emmerich, N. P., Bachor, O. A. & Stevanovic, S. (1999) *Immunogenetics* 50, 213-219.
- 12.: Drexler, I., Antunes, E., Schmitz, M., Wolfel, T., Huber, C., Erfle, V., Rieber, P., Theobald, M. & Sutter, G. (1999) *Cancer Res.* 59, 4955-4963.
- 13.: Pascolo, S., Bervas, N., Ure, J. M., Smith, A. G., Lemonnier, F. A. & Perarnau, B. (1997) *J. Exp. Med.* 185, 2043-2051.
- 14.: Alcami, A. & Smith, G. L. (1996) *Proc. Natl. Acad. Sci.* USA 93, 11029-11034.
- 15.: Goebel, S. J., Johnson, G. P., Perkus, M. E., Davis, S. W., Winslow, J. P. & Paoletti, E. (1990) *Virology* 179, 247-266.
- 16.: Massung, R. F., Liu, L.-I., Qi, J., Knight, J. C., Yuran, T. E., Kerlavage, A. R., Parsons, J. M., Venter, J. C. & Esposito, J. J. (1994) Virology 201, 215-240.
- 17.: Rodriguez, J. F., Paez, E. & Esteban, M. (1987) *J. Virol.* 61, 395-404.
- 18.: Zinoviev, V. V., Tchikaev, N. A., Chertov, O. Y. & Malygin, E. G. (1994) *Gene* 147, 209-214.
- 19.: Bronte, V., Carroll, M. W., Goletz, T. J., Wang, M., Overwijk, W. W., Marincola, F., Rosenberg, S. A., Moss, B. & Restifo, N. P. (1997) *Proc. Natl.*
*Acad. Sci. USA* 94, 3183-3188.
- 20.: da Fonseca, F. G., Wolffe, E. J., Weisberg, A. & Moss, B. (2000) *J*. *Virol.* 74, 7508-7517.
- 21.: Lee, H. J., Essani, K. & Smith, G. L. (2001) *Virology* 281, 170-192.
- 22.: Herrlich, A. & Mayr, A. (1957) *Arch. Virusforsch.* 7, 284-296.
- 23.: Mayr, A., Hochstein-Mintzel, V. & Stickl, H. (1975) *Infection* 3, 6-14.
- 24.: Sutter, G. & Moss, B. (1992) *Proc. Natl. Acad. Sci. USA* 89, 10847-10851.
- 25.: Sutter, G., Wyatt, L. S., Foley, P. L., Bennink, J. R. & Moss, B. (1994) *Vaccine* 12, 1032-1040.
- 26.: Meyer, H., Sutter, G. & Mayr, A. (1991) *J. Gen. Virol.* 72, 1031-1038.
- 27.: Lee, M., Roos, J., McGuigan, L., Smith, K., Cormier, N., Cohen, L., Roberts, B. & Payne, L. (1992) *J. Virol.* 66, 2617-2630.
- 28.: Sahin, U., Türeci, Ö., Schmitt, H., Cochlovius, B., Johannes, T., Schmits, R., Stenner, F., Luo, G., Schobert, 1. & Pfreundschuh, M. (1995) *Proc. Natl. Acad. Sci. USA* 92, 11810-11813.
- 29.: Benenson, A. S., Cherry, J. D., McIntosh, K., Connor, J. D., Alling, D. W., Nakano, J., Rolfe, U. T., Schanberger, J. E., Todd, W. A., DeCastro, F., Horvath, F. L., Bairan, A., Phillips, 1. A., Galasso, G. J. & Matthels, M. J. (1977) *J. Infect. Dis.* 135, 135-144.
- 30.: Hirsch, V., Fuerst, T., Sutter, G., Carroll, M., Yang, L., Goldstein, S., Piatak, M., Elkins, W., Alvord, G., Montefiori, D., Moss, B. & Lifson, J. (1996) *J. Virol.* 70, 3741-3752.

## Claims

1. An immunogenic Orthopoxvirus antigen comprising at least one of the peptides of SEQ ID NO: 1-50 or variants thereof, wherein said variants comprise one or more additions, insertions, substitutions and/or deletions as compared to the respective peptide of SEQ ID NO: 1-50, and wherein the immunogenic activity of the variant is substantially equal to the activity of the respective unmodified peptide of SEQ ID NO: 1-50.

2. An isolated nucleic acid, which codes for one or more of the peptide of claim 1.

3. An isolated nucleic acid, which is a transcriptional product of the nucleic acid of claim 2.

4. An isolated nucleic acid, which selectively hybridizes to the nucleic acids of claims 2 or 3 under moderately stringent conditions.

5. An immunogenic antigen, which is encoded by a nucleic acid of one of claims 2-4.

6. A vector, which comprises one or more of the nucleic acid sequences of any of claims 2-4.

7. An expression vector, which comprises one or more of the nucleic acid sequences of any of claims 2-4 and one or more regulatory sequences.

8. The vector of claim 6 or 7, which is a plasmid.

9. A host cell, which has been transformed with the vector of any of claims 6-8.

10. The host cell of claim 9, which is a eukaryotic cell.

11. The host cell of claim 10, which is a mammalian cell, plant cell, yeast cell or an insect cell.

12. The mammalian cell of claim 11, which is a CHO, COS, HeLa, 293T, HEH or BHK cell.

13. The host cell of claim 9, which is a prokaryotic cell.

14. The host cell of claim 13, which is *E.coli* or *Bacillus subtilis*.

15. An antigen presenting cell, which has been pulsed with one or more of the peptides of claim 1.

16. The antigen presenting cell of claim 15, which is a dendritic cell or a B cell.

17. A diagnostic composition comprising one or more of the peptides of claim 1 and/or an antigen presenting cell of claim 15 or 16.

18. A diagnostic kit for detecting an immune response induced by smallpox vaccines or Orthopoxvirus vector vaccines comprising at least one or more peptides according to claim 1 or an antigen presenting cell of claim 15 or 16.

19. A pharmaceutical composition comprising a therapeutically effective amount of one or more peptides of claim 1, one or more of the nucleic acids of claims 2-4 or a vector of claim 6-8, and a pharmaceutically acceptable carrier.

20. The pharmaceutical composition of claim 19, which is a vaccine.

21. Use of the pharmaceutical composition of claim 19 or 20 in the protective immunization against a smallpox infection in a mammal, preferably a human.

22. An ex vivo-method of detecting a T cell response induced by smallpox vaccines or Orthopoxvirus vector vaccines in a mammal, preferably a human patient, comprising the following steps:
a) providing a sample containing T cells from a patient, which patient has been immunized with a smallpox vaccine or Orthopoxvirus vector vaccine, and
b) co-cultivating said sample with one or more peptides of claim 1 or a diagnostic composition of claim 17, under conditions which allow an activation of said T cells, and
c) determining any specific activity of T cells contained in that sample against any peptide indicated in b).

23. The method of claim 22, wherein the T cells are CD 8+ T cells.

24. The method of claim 22 or 23, wherein the determination in step c) is performed by a ⁵¹Cr-release assay.

25. The method of claim 22 or 23, wherein the determination in step c) is performed by measuring the amount of cytokines in said T cells.

26. The method of claim 25, wherein the cytokines are measured by intracellular cytokine staining.

27. The method of claim 25, wherein the cytokines are measured by an ELISPOT assay.

28. The method of one or more of claims 22-27, wherein the sample is a human body fluid, preferably a blood sample.


SEQUENCE LISTING

<110>  GSF Forschungszentrum für Umwelt und Gesundheit GmbH

<120>  Orthopoxvirus antigens and use thereof

<130>  P14625

<160>  50

<170>  PatentIn version 3.1

<210>  1
<211>  10
<212>  PRT
<213>  Vaccinia virus

<400>  1

Ile Ile Ala Ile Val Phe Val Phe Ile Leu
1               5                   10


<210>  2
<211>  10
<212>  PRT
<213>  Vaccinia virus

<400>  2

Tyr Leu Asp Asn Leu Leu Pro Ser Thr Leu
1               5                   10


<210>  3
<211>  9
<212>  PRT
<213>  Vaccinia virus

<400>  3

Phe Ile Leu Thr Ala Ile Leu Phe Leu
1               5


<210>  4
<211>  9
<212>  PRT
<213>  Vaccinia virus

<400>  4

Tyr Leu Asp Asn Leu Leu Pro Ser Thr
1               5


<210>  5
<211>  9
<212>  PRT
<213>  Vaccinia virus

<400>  5

Gly Leu Ile Ile Ile Ser Ile Phe Leu
1               5

<210>  6
<211>  9
<212>  PRT
<213>  Vaccinia virus

<400>  6

Ile Ile Ile Ser Ile Phe Leu Gln Val
1               5

<210>  7
<211>  9
<212>  PRT
<213>  Vaccinia virus

<400>  7

Phe Ala Ile Ile Ala Ile Val Phe Val
1               5

<210>  8
<211>  9
<212>  PRT
<213>  Vaccinia virus

<400>  8

Tyr Leu Phe Arg Ile Val Ser Thr Val
1               5

<210>  9
<211>  9
<212>  PRT
<213>  Vaccinia virus

<400>  9

Leu Leu Asn Asn Ser Leu Gly Ser Val
1               5

<210>  10
<211>  9
<212>  PRT
<213>  Vaccinia virus

<400>  10

Asn Leu Ala Thr Ser Ile Tyr Thr Ile
1               5

<210>  11
<211>  9
<212>  PRT
<213>  Vaccinia virus

<400> 11

Asp Leu Leu Asn Phe Met Val Ser Val
1               5

<210> 12
<211> 9
<212> PRT
<213> Vaccinia virus

<400> 12

Leu Ile Arg Pro Lys Ile Leu Ser Met
1               5

<210> 13
<211> 10
<212> PRT
<213> Vaccinia virus

<400> 13

Ile Leu Leu Asn Asn Ser Leu Gly Ser Val
1               5                   10

<210> 14
<211> 10
<212> PRT
<213> Vaccinia virus

<400> 14

Tyr Leu Ile Asp Thr Thr Ser Arg Glu Leu
1               5                   10

<210> 15
<211> 10
<212> PRT
<213> Vaccinia virus

<400> 15

Met Leu Val Ser Gln Ala Leu Asn Ser Val
1               5                   10

<210> 16
<211> 10
<212> PRT
<213> Vaccinia virus

<400> 16

Met Met Pro Ile Lys Ser Ile Val Thr Leu
1               5                   10

<210> 17

<211>  10
<212>  PRT
<213>  Vaccinia virus

<400>  17

Ser Leu Ile Arg Pro Lys Ile Leu Ser Met
1               5                   10


<210>  18
<211>  9
<212>  PRT
<213>  Vaccinia virus

<400>  18

Ser Leu Ser Ala Tyr Ile Ile Arg Val
1               5


<210>  19
<211>  9
<212>  PRT
<213>  Vaccinia virus

<400>  19

Ile Met Phe Met Leu Ile Phe Asn Val
1               5


<210>  20
<211>  9
<212>  PRT
<213>  Vaccinia virus

<400>  20

Tyr Ile Ile Arg Val Thr Thr Ala Leu
1               5


<210>  21
<211>  9
<212>  PRT
<213>  Vaccinia virus

<400>  21

Asn Val Ile Gly Leu Ile Val Ile Leu
1               5


<210>  22
<211>  9
<212>  PRT
<213>  Vaccinia virus

<400>  22

Tyr Thr Gly Gly Tyr Asp Val Ser Leu
1               5

<210> 23
<211> 10
<212> PRT
<213> Vaccinia virus

<400> 23

Gly Leu Phe Asp Ile Asn Val Ile Gly Leu
1               5                   10


<210> 24
<211> 10
<212> PRT
<213> Vaccinia virus

<400> 24

Ala Met His Asp Lys Lys Ile Asp Ile Leu
1               5                   10


<210> 25
<211> 9
<212> PRT
<213> Vaccinia virus

<400> 25

Arg Leu Tyr Lys Pro Leu Leu Lys Leu
1               5


<210> 26
<211> 9
<212> PRT
<213> Vaccinia virus

<400> 26

Ala Leu Phe Tyr Lys Asp Gly Lys Leu
1               5


<210> 27
<211> 9
<212> PRT
<213> Vaccinia virus

<400> 27

Phe Leu Ser Ile Val Val Asp His Val
1               5


<210> 28
<211> 9
<212> PRT
<213> Vaccinia virus

<400> 28

Arg Ile Phe Val Arg Val Tyr Asn Val
1               5

<210>  29
<211>  9
<212>  PRT
<213>  Vaccinia virus

<400>  29

Leu Thr Ile Lys Gln Thr Ala Glu Val
1               5

<210>  30
<211>  9
<212>  PRT
<213>  Vaccinia virus

<400>  30

Phe Leu Tyr Asn Phe Trp Thr Asn Val
1               5

<210>  31
<211>  9
<212>  PRT
<213>  Vaccinia virus

<400>  31

Thr Leu Phe Pro Gly Asp Asp Asp Leu
1               5

<210>  32
<211>  9
<212>  PRT
<213>  Vaccinia virus

<400>  32

Asn Leu Glu Lys Lys Ile Thr Asn Val
1               5

<210>  33
<211>  9
<212>  PRT
<213>  Vaccinia virus

<400>  33

Thr Leu Lys Gln Arg Leu Thr Asn Leu
1               5

<210>  34
<211>  9
<212>  PRT

<213>  Vaccinia virus

<400>  34

Thr Leu Arg Ala Ala Met Ile Ser Leu
1               5


<210>  35
<211>  9
<212>  PRT
<213>  Vaccinia virus

<400>  35

Ala Met Ile Ser Leu Ala Lys Lys Ile
1               5


<210>  36
<211>  10
<212>  PRT
<213>  Vaccinia virus

<400>  36

Ala Met Ala Asp Val Ile Ile Asp Asp Val
1               5                   10


<210>  37
<211>  9
<212>  PRT
<213>  Vaccinia virus

<400>  37

Ile Val Cys Glu Ala Ile Lys Thr Ile
1               5


<210>  38
<211>  9
<212>  PRT
<213>  Vaccinia virus

<400>  38

Ala Leu Tyr Asp Leu Gln Arg Ser Ala
1               5


<210>  39
<211>  9
<212>  PRT
<213>  Vaccinia virus

<400>  39

Ala Ala Lys Leu Ala Val Asp Lys Leu
1               5

<210>  40
<211>  9
<212>  PRT
<213>  Vaccinia virus

<400>  40

Ala Val Asp Lys Leu Leu Gly Tyr Val
1               5


<210>  41
<211>  10
<212>  PRT
<213>  Vaccinia virus

<400>  41

Tyr Ile Asp Glu Arg Ser Asn Ala Glu Ile
1               5                   10


<210>  42
<211>  9
<212>  PRT
<213>  Vaccinia virus

<400>  42

Gln Leu Asn Met Glu Lys Arg Glu Val
1               5


<210>  43
<211>  9
<212>  PRT
<213>  Vaccinia virus

<400>  43

Leu Leu Phe Ile Pro Asp Ile Lys Leu
1               5


<210>  44
<211>  9
<212>  PRT
<213>  Vaccinia virus

<400>  44

Val Met Leu Asn Asp Val Ser Ile Val
1               5


<210>  45
<211>  9
<212>  PRT
<213>  Vaccinia virus

<400>  45

Ile Leu Leu Ser Cys Ile His Thr Thr

1                   5


<210>  46
<211>  9
<212>  PRT
<213>  Vaccinia virus

<400>  46

Ala Ile Ala Asp Asn Asn Val Arg Leu
1                   5


<210>  47
<211>  9
<212>  PRT
<213>  Vaccinia virus

<400>  47

Asp Leu Ala Ile Leu Leu Ser Cys Ile
1                   5


<210>  48
<211>  9
<212>  PRT
<213>  Vaccinia virus

<400>  48

Val Ile Tyr Glu Ala Val Ile His Leu
1                   5


<210>  49
<211>  9
<212>  PRT
<213>  Vaccinia virus

<400>  49

Ile Ile Ile Asn Gly Asp Ile Ala Leu
1                   5


<210>  50
<211>  9
<212>  PRT
<213>  Vaccinia virus

<400>  50

Asn Met Ile Ser Lys Lys Tyr Lys Val
1                   5